# EUROPEAN PATENT SPECIFICATION

(11) **EP 1 850 888 B1**
(45) Date of publication and mention of the grant of the patent: **17.09.2008**
(21) Application number: 06704705.0
(22) Date of filing: 20.02.2006
(51) Int. Cl.: A61L 12/14, A61L 12/10, C11D 3/00

(54) **Contact lens care composition**
PFLEGEZUSAMMENSETZUNG FÜR KONTAKTLINSEN
Composition de soins pour lentilles de contact

(30) Priority: 23.02.2005 AT 3052005
(43) Date of publication of application: 07.11.2007
(73) Proprietor: Obwaller, Andreas, 1100 Vienna (AT)
(72) Inventor: Obwaller, Andreas, 1100 Vienna (AT)
(74) Representative: Sonn & Partner Patentanwälte
(86) International application number: PCT/AT2006/000063
(87) International publication number: WO 2006/089320

(56) References cited:
- EP-A- 0 381 617
- EP-A- 0 819 968
- WO-A-94/16743
- DE-A1- 4 132 345
- DE-A1- 10 203 195
- DE-A1- 10 313 272
- WALOCHNIK J ET AL: "CYTOTOXIC ACTIVITIES OF ALKYLPHOSPHOCHOLINES AGAINST CLINICAL ISOLATES OF ACANTHAMOEBA SPP" ANTIMICROBIAL AGENTS AND CHEMOTHERAPY, AMERICAN SOCIETY FOR MICROBIOLOGY, WASHINGTON, DC, US, vol. 46, no. 3, March 2002 (2002-03), pages 695-701, XP001146164 ISSN: 0066-4804 cited in the application

## Description

The present invention relates to compositions with enhanced antimicrobial efficacy against Acanthamoeba.

Acanthamoeba keratitis is a corneal disease predominantly associated with contact lens wear. The occurrence of acanthamoeba keratitis has been rising since 1990 in correlation to the enhanced number of contact lens wearers. Approximately 3000 cases of acanthamoeba keratitis have been reported around the world. The disease usually progresses slowly, however, in most cases it ends up in a fulminant infection, very often leading to severe loss of vision and sometimes even to the enucle-ation of the afflicted eye. Meanwhile, Acanthamoeba are, besides the pseudomonads, the most common causative agents of contact lens-associated keratitis. It is estimated that the annual incidence of acanthamoeba keratitis in the USA is 1:250,000 inhabitants. In Europe most cases have been documented in the UK (around 400).

Contaminated contact lenses, lens cases or contact lens care systems usually are the first step in an acanthamoeba keratitis pathogenesis. Evidence of the ease with which human infection can potentially occur is reflected by the isolation of Acanthamoeba from water drawn from bathroom taps and from dust around a washbasin. Since Acanthamoeba species are ubiquitous in the environment, lens care systems could possibly even become contaminated with dust from the air. The presence of bacteria in contact lens cases and solutions originating from tap water or elsewhere predisposes to ocular Acanthamoeba infection. The amoeba feed on bacteria and multiply, with the result that large potential amoebic inocula may be present.

Several studies have shown that the effect of disinfectants against Acanthamoeba is insufficient and varies between different strains. Development of resistances and amoebostatic rather than amoebicidic effects are counter indicators of current drugs. Thus, a need for compositions for inhibiting growth of protozoans remains, such as Acanthamoeba in or on eye care products such as contact lenses, contact lens solutions and contact lens cases, in order to reduce the incidence of acanthamoebic keratitis and other ophthalmic pathologies due to the presence of protozoans. Current drugs administered in cases of acanthamoeba keratitis include propamidine isethionate, chlorhexidine gluconate and polyhexamethylene biguanide (Schuster et al., Cur. Treatm. Opt. Inf. Dis. 5:273-282, 2003).

General disinfecting contact lens care compositions are known in the state of the art and are disclosed e.g. in the WO 04/058930 A1. Such general disinfectants have been tested (see examples) and proven to be ineffective against Acanthamoeba contaminations on contact lenses.

The WO 2004/030710 A1 describes ophthalmic compositions for use as eye drop or contact lens cleaning solutions which comprise antimicrobial polycationic materials. Such polycationic materials are for example cationic cellulose derivatives with polyvalent cation chelating moieties, for example quaternary amino groups, which effectively inhibit protozoan cell function, particularly cell growth.

The GB 2 329 126 A describes disinfectant solutions containing polyhexamethylene biguanides.

The DE 102 03 195 A1 describes solutions of miltefosine and pharmaceutical compositions of miltefosine for oral application to treat diseases caused by protozoa, e.g. Leishmaniase.

The DE 41 32 345 A1 describes a composition of miltefosine for peroral or intravenous and intramuscular application.

The DE 103 13 272 A1 discloses the use of miltefosine for the topical treatment of skin cancer.

The WO 94/16743 describes a contact lens care solution to eliminate hydrogen peroxide resistant microorganisms with hydrogen peroxide wherein the resistance is obviated by lysozyme which can degrade the protecting cellulose or chitin.

The EP 0 381 617 A describes a contact lens care solution with a glycol and a tonicity component.

The EP 0 819 968 A describes further glycol containing disinfection solutions which additionally comprise hydrogen peroxide.

Silvany et al. (Ophthalmology 97(3) 286-90 (abstract), 1990) analysed contact lens disinfectants against Acanthamoeba. The most effective compound under investigation was chlorhexidine.

The GB 2 333 609 A describes a solution of sodium salicylate which inhibits the attachment of Acanthamoeba to contact lenses.

Furthermore, potential ocular drugs or preservatives have to fulfill certain criteria, e.g. deleterious effects on the tear film stability have to be avoided. The tear film is a three-layered fluid of 7 µm thickness composed of lipid, aqueous and mucin phases (Holly et al., Exp. Eye Res. 15:515-525, 1973). Any discontinuity and instability of this tear film over the cornea may lead to ocular non-wetting tears disorders called dry eye. Tear disorders are assessed by different clinical tests including break-up time test, the Schirmer test, fluorescein and rose bengal staining and biomicroscopy (Stulting et al. in Leibowitz, Corneal Disorders, Clinical Diagnosis and Management, W.B. Saun-ders, Philadelphia, PA, pp. 445-468, 1984). Especially surface-active agents could solubilize the lipid layer of the tear film, which would lead to tear film rupture (Furrer et al., Eu. J. Pharm. Bioph., 53:262-280, 2002).

In a recent study it has been shown that miltefosine (1-hexadecylphosphorylcholine, also known as hexadecylphosphocholine, also referred to as HePC) shows high amoebicidal activity against trophozoites and cysts of three strains of Acanthamoeba of different pathogenicity. The treatment with miltefosine resulted in vacuolization, rounding up of cells, blebbing, and finally complete lysis of the cells after less than 30 minutes. (Walochnik et al., Antimicrob. Agents Chemoth-er. 46: 695-701, 2002). Commercially available multi-purpose solutions, however, showed only (minor) reactivity against trophozoites and not against cysts (Hiti et al. Br J Ophthalmol. 86: 144-6, 2002).

Miltefosine is a detergent-like drug with both ionic and lipophilic characteristics. It is an ester of phosphorylcholine and hexadecanol and therefore resembles a membrane-active al-kylphospholipid. It is an inhibitor of the CTP phosphocholine cytidylyl transferase and has antimetastatic properties. Additionally, it is a very effective drug against visceral infections of Leishmania. Leishmania have high levels of ether-lipids and miltefosine is believed to act on key enzymes involved in the metabolism of ether lipids (More et al., J. Postgrad. Med. 49:101-103, 2003). Leishmaniasis is a very virulent tropical disease and transmitted by sand flies. Symptoms of visceral leishmaniasis include fever, spleen and liver enlargement, blood deficiencies, bleeding of mucous membranes and severe weight loss. Cutaneous leishmaniasis, although not lethal, is a severely disfiguring condition. Miltefosine (Impavido^{®}) is licensed in most states for the oral treatment of leishmaniasis.

The technical problem underlying the present invention is to provide means and methods which allow the efficient prophylaxis against protozoan eye infections, especially in the field of contact lens care. A special goal of the present invention is to provide means to prevent Acanthamoeba infections transmitted through contact lens wear.

It is based on the use of hexadecylphosphocholine to eliminate the Acanthamoeba exactly at their major route into the human eye before they can do any damage. It contributes to saving health care cost by exercising prophylaxix rather than treatment.

Although the efficacy of miltefosine against Acanthamoeba is known in the prior art, practical uses of this compound have been restricted for the oral treatment of leishmaniasis so far. Factors like lipophilicity and surface-active properties of miltefosine appear to have been counter-indicators for the use in a composition as presented herein.

Surprisingly, the composition of the present invention did not exhibit negative effects, which allows the use in an ophthalmic composition or lens care system with amazing efficiency against protozoan germs such as Acanthamoeba.

The present invention provides contact lens care compositions containing miltefosine as antimicrobial agent in an amount effective for disinfecting contact lenses. Miltefosine therein is the primary amoebicidal substance, preferably used for eradication of Acanthamoeba and protozoan organisms from contact lenses. Preferably, the Acanthamoeba strains belong to A. cas-tellani, A. hachetti and A. polyphaga.

Compositions for use in connection with the eye and with contact lenses which are subsequently placed in the eye are desirable such as to avoid discomfort and damage to the eye. Certain antibacterial compositions currently in use employ bactericides which, if used in connection with hydrophilic soft contact lenses, may be absorbed and concentrated in the lens. Such a lens, if placed in the eye, can release a concentrated solution leading to irritation of the eye. Therefore, ophthalmic compositions consist of substances, which can mediate effects of or alter the solubility of the specific antimicrobial substances employed. Usual additional substances in ophthalmic compositions are buffers, surfactants, viscosity increasing components, tonicity components and chelating agents.

The present invention can be used with all contact lenses such as conventional hard, soft, rigid and soft gas permeable lenses, however use of the present invention in soft lens case systems being especially advantageous. The compositions of the present invention may be aqueous or non-aqueous. Aqueous solutions are preferred. The composition according to the invention can also be pressed in a tablet form. Before use, the tablets can be dissolved in water or any other solution used for contact lens care systems. If the composition is provided in solid form, all concentrations given in the specification and the claims of the present invention refer to concentrations the compounds of the composition have after appropriate solvation.

The concentration of miltefosine in a preferred embodiments of the composition of the present invention is between 5 and 500µM, further preferred between 10 and 300 µM, even further preferred between 20 and 200 µM.

A preferred implementation of the composition according to the present invention further comprises buffer components (phosphate salts or other buffering salts, such as TRIS) in an amount effective in maintaining the pH of the solution within a physiologically acceptable range, preferably with a pH between 6 and 8.

The composition may also comprise a surfactant in an amount effective in cleaning a contact lens. Examples of surfactants are poloxamines, poloxamers, alkyl ethoxylates, alkyl phenyl ethoxylates or other non-ionic, anionic and dipolar ionic surfactants known in the art.

A further implementation of the composition of the present invention additionally comprises a viscosity increasing component, preferably selected from the group of cellulose derivatives, polyols, polyethylene oxide (PEO) containing polymers, polyvinyl alcohol and polyvinyl pyrrolidone. Cellulose derivatives are for example cationic cellulose polymers, hydroxypropyl methylcellulose, hydroxyethylcellulose and methylcellulose. An example of a polyol is polyethylene glycol.

The composition of the present invention furthermore preferably comprises a tonicity component, preferably salts, such as sodium chloride, potassium chloride and calcium chloride, or non-ionic tonicity agents, preferably propylene glycol or glycerol. Practical tonicities are in a preferred range of 200 - 600 mOsm, in an even more preferred range between 250 - 450 mOsm.

A further implementation of the composition of the present invention additionally comprises a chelating component, preferably ethylenediaminetetraacetic acid, alkali metal salts of ethylenediaminetetraacetic acid or mixtures thereof.

A further implementation of the composition of the present invention additionally comprises a protease. Proteases (enzymatic cleaners) are especially useful for contact lens wearers susceptible to high levels of protein deposits.

A further implementation of the composition of the present invention additionally comprises further disinfectants. An additional disinfectant, beside miltefosine, can be employed as functional preservative, but it may also function to potentate, complement or broaden the spectrum of the microbiocidal activity of another germicide. Suitable antimicrobial components are those generally employed in ophthalmic applications and include, but are not limited to, quaternary ammonium salts used in ophthalmic applications such as poly[dimethylimino-2-butene-1,4-diyl] chloride, alpha-[4-tris(2-hydroxyethyl) ammonium]-dichloride, benzalkonium halides, and biguanides, such as salts of alexidine, alexidine-free base, salts of chlorhexidine, hexamethylene biguanides and their polymers, and salts thereof, antimicrobial polypeptides, chlorine dioxide precursors, and the like and mixtures thereof. Generally, the hexamethylene biguanide polymers (PHMB), also referred to as polyaminopropyl biguanide (PAPB), is preferred.

As mentioned above, the composition of the present invention can be provided in solid state. Therefore, the present invention provides a miltefosine containing composition, for the cleaning of contact lenses, pressed in a tablet form.

The present invention encompasses the use of a composition containing miltefosine for cleaning, chemical disinfection, storing or rinsing contact lenses.

A further use of a composition according to the present invention relates to the elimination of protozoan organisms from a contact lens.

Preferably the composition of the present invention is used for the elimination of Acanthamoeba from a contact lens.

The present invention furthermore comprises the use of the present contact lens care composition, wherein the contact lens is selected from hard, soft, rigid or soft gas permeable lenses, preferably soft lenses.

The composition of the present invention can be used as storing solution for contact lenses in order to prevent contaminations of Acanthamoeba and other protozoan organisms. Therefore, the present invention encompasses the method for the purification of contact lenses, wherein the contact lens is stored in the composition according to the present invention for at least 1 minute.

A preferred implementation of the invention is a "multi-purpose solution" containing the composition of the present invention. The term "multi-purpose solution" means that the solution may be daily or periodically used for cleaning, chemical disinfection, storing, and rinsing contact lenses.

The present invention also comprises a "multi-purpose solution system" or "multi-purpose solution package" or a "kit", which comprises the composition of the present invention together with rinsing solutions and/or drop delivery tools, preferably pipettes. Rinsing solutions can be appropriate sterilized buffer solutions or artificial tears. Such a kit may contain the composition of the present invention in packages (tablets or solution packages) indicated for the daily usage.

As an illustration of the present invention, several examples are provided below. These examples serve only to further illustrate aspects of the invention and should not be construed as limiting the invention.

The activity of various multi-purpose contact lens solutions (for cleaning, rinsing, storing and disinfecting a contact lens while the lens is not worn) was tested against cysts of A. castellani.

### EXAMPLES

### Example 1: Miltefosine and contact lens solutions

Contact lens disinfection solutions tested (Table 1) were purchased from local retail stores. In case of the H₂O₂ based systems,neutralization of the 3% hydrogen peroxide is achieved within 6 hours by a catalytic platinum disk which is located in the contact lens storage case according to the manufacturer's instructions. Miltefosine was dissolved in 5% Ethanol (2mM stock solution) and tested at 80 µM in Acanthamoeba medium (PYG) or in contact lens disinfection medium.

### Example 2: Acanthamoeba cyst production for susceptibility tests

A. castellanii, was isolated from a patient suffering from a severe Acanthamoeba keratitis. Isolation was achieved by inoculating corneal epithelium onto non-nutrient agar plates covered with a 48-h-old culture of Escherichia coli. The isolate was cloned by transferring a single cyst onto a fresh plate with a micromanipulator. Axenisation was achieved by harvesting cysts from parallel cultures and incubating them in 3% HCl overnight in order to eliminate coexisting bacteria. The cysts were then transferred to sterile filtrated PYG (proteose peptone-yeast extract-glucose) that was used as axenic medium further on. Synchronized encystment of the amoebae was achieved by long term storage without the addition of fresh medium. The process of encystment including morphological changes of the cyst wall was observed daily by phase-contrast microscopy. After 14 days the cysts of both strains were in their mature stage. The cysts were harvested by centrifugation (500 g/ 7 minutes), resuspended in sterile 0.9% NaCl and counted in a Burker- Turk haemocytometer. Of each strain two suspensions were prepared, one with 10⁴ and one with 10⁸ cysts/ ml, respectively.

### Example 3: Performance of the susceptibility tests for Acanthamoeba cysts

Tests were performed in 15 ml centrifugation tubes. 100 µl of the respective cyst suspension (10³ cells) and 8 ml of the respective contact lens solution were added per tube. All solutions were used according to the manufacturers' instructions. After a soaking time of 8 hours the pellets (after 500g/ 7 minutes centrifugation) of the respective tubes were inoculated onto non-nutrient agar plates covered with a layer of Escherichia coli. The plate cultures were sealed and incubated at 30°C for 14 days. Amoebic growth was observed daily by phase contrast microscopy. All experiments were carried out in triplicate. The control groups were performed with sterile 0.9% NaCl.

**Table 1: Viable cells of Acanthamoeba detected on non-nutrient agar plates after incubation with four commercially available contact lens care solutions comprising H₂O₂ (1, 2) Polyhexamethylene Biguanide (3), sorbitol (4) and Bactofongus (5), Miltefosine and contact lens care solutions comprising Miltefosine respectively, after 8 hours (+, viable cells; -, no viable cells).**

| | Viable Acanthamoeba cells after disinfection with commercially available disinfectants | Viable Acanthamoeba cells after disinfection with commercially available disinfectants comprising 80µM Miltefosine |
|---|---|---|
| 1 | + | - |
| 2 | + | - |
| 3 | + | - |
| 4 | + | - |
| 5 | + | - |
| Miltefosine | - | |
| 9% NaCl | + | - |

## Claims

1. Use of miltefosine as antimicrobial agent in a contact lens care composition for disinfecting contact lenses.

2. Use according to claim 1, wherein miltefosine is in an amount of 5 - 500 µM in the composition.

3. Use according to claim 1 or 2, wherein miltefosine is in an amount of 20 - 200 µM in the composition.

4. Use according to any one of claims 1 to 3, **characterized in that** the composition comprises a buffer component, preferably a phosphate buffer component.

5. Use according to any one of claims 1 to 4, wherein the pH of the solution of the composition is in a range of 6 - 8.

6. Use according to any one of claims 1 to 5, **characterized in that** the composition additionally comprises a surfactant.

7. Use according to any one of claims 1 to 6, **characterized in that** the composition additionally comprises a viscosity increasing component, preferably selected from the group of cellulose derivatives, polyols, polyethylene oxide (PEO) containing polymers, polyvinyl alcohol and polyvinyl pyrrolidone.

8. Use according to any one of claims 1 to 7, **characterized in that** the composition comprises a tonicity component, preferably salts, such as sodium chloride, potassium chloride and calcium chloride, or non-ionic tonicity agents, preferably propylene glycol or glycerol.

9. Use according to any one of claims 1 to 8, **characterized in that** the composition comprises a chelating component, preferably ethylenediaminetetraacetic acid, alkali metal salts of ethylenediaminetetraacetic acid or mixtures thereof.

10. Use according to any one of claims 1 to 9, **characterized in that** the composition additionally comprises a protease.

11. Use according to any one of claims 1 to 10, **characterized in that** the composition additionally comprises further disinfectants.

12. Use according to any one of claims 1 to 11, **characterized in that** the composition is pressed in a tablet form.

13. Use of a composition as defined in any one of claims 1 to 12 for cleaning, chemical disinfection, storing, and/or rinsing contact lenses.

14. Use according to claim 13 for the elimination of protozoan organisms from a contact lens.

15. Use according to claim 14 for the elimination of Acanthamoeba from a contact lens.

16. Use according to any one of claims 1 to 15, wherein the contact lens is selected from hard, soft, rigid or soft gas permeable lenses, preferably soft lenses.

17. Method for the purification of contact lenses wherein the contact lens is stored in the composition as defined in any one of claims 1 to 12 for at least 1 minute.

18. Use of a composition as defined in any one of claims 1 to 12 for daily or periodical cleaning, chemical disinfection, storage and rinsing of contact lenses.

19. Contact lens care composition comprising miltefosine as antimicrobial agent for disinfecting contact lenses, preferably in an amount of 5 - 500 µM, in particular 20 - 200 µM, and a protease, wherein the composition is preferably further defined as in any one of claims 4 to 9, 11 and 12.

20. Kit containing a composition according to claim 19 , rinsing solutions and/or drop delivery tools, preferably pipettes.

## Patentansprüche

1. Verwendung von Miltefosin als antimikrobielles Mittel in einer Kontaktlinsen-Pflegezusammensetzung zur Desinfektion von Kontaktlinsen.

2. Verwendung nach Anspruch 1, wobei Miltefosin in einer Menge von 5-500 µM in der Zusammensetzung vorliegt.

3. Verwendung nach Anspruch 1 oder 2, wobei Miltefosin in einer Menge von 20-200 µM in der Zusammensetzung vorliegt.

4. Verwendung nach einem der Ansprüche 1 bis 3, **dadurch gekennzeichnet, dass** die Zusammensetzung eine Puffer-Komponente, vorzugsweise eine Phosphatpuffer-Komponente, aufweist.

5. Verwendung nach einem der Ansprüche 1 bis 4, wobei der pH-Wert der Lösung der Zusammensetzung in einem Bereich von 6-8 liegt.

6. Verwendung nach einem der Ansprüche 1 bis 5, **dadurch gekennzeichnet, dass** die Zusammensetzung zusätzlich ein oberflächenaktives Mittel umfasst.

7. Verwendung nach einem der Ansprüche 1 bis 6, **dadurch gekennzeichnet, dass** die Zusammensetzung zusätzlich eine Viskositäts-erhöhende Komponente, vorzugsweise ausgewählt aus der Gruppe der Cellulosederivate, Polyole, Polyethylenoxid(PEO)-hältigen Polymere, Polyvinylalkohol und Polyvinylpyrrolidon, umfasst.

8. Verwendung nach einem der Ansprüche 1 bis 7, **dadurch gekennzeichnet, dass** die Zusammensetzung eine Tonizitäts-Komponente, vorzugsweise Salze, wie Natriumchlorid, Kaliumchlorid und Calciumchlorid, oder nicht-ionische Tonizitätsmittel, vorzugsweise Propylenglykol oder Glycerin, umfasst.

9. Verwendung nach einem der Ansprüche 1 bis 8, **dadurch gekennzeichnet, dass** die Zusammensetzung eine Chelatbildner-Komponente, vorzugsweise Ethylendiamintetraessigsäure, Alkalimetallsalze von Ethylendiamintetraessigsäure, oder Mischungen davon, umfasst.

10. Verwendung nach einem der Ansprüche 1 bis 9, **dadurch gekennzeichnet, dass** die Zusammensetzung zusätzlich eine Protease umfasst.

11. Verwendung nach einem der Ansprüche 1 bis 10, **dadurch gekennzeichnet, dass** die Zusammensetzung zusätzlich weitere Desinfektionsmittel umfasst.

12. Verwendung nach einem der Ansprüche 1 bis 11, **dadurch gekennzeichnet, dass** die Zusammensetzung in Tablettenform gepresst ist.

13. Verwendung einer Zusammensetzung nach einem der Ansprüche 1 bis 12 zur Reinigung, chemischen Desinfektion, Aufbewahrung und/oder Spülung von Kontaktlinsen.

14. Verwendung nach Anspruch 13 zur Eliminierung von protozoischen Organismen von einer Kontaktlinse.

15. Verwendung nach Anspruch 14 zur Eliminierung von Acanthamoeba von einer Kontaktlinse.

16. Verwendung nach einem der Ansprüche 1 bis 15, wobei die Kontaktlinse ausgewählt ist aus harten, weichen, starren oder weichen gasdurchlässigen Linsen, vorzugsweise weichen Linsen.

17. Verfahren zur Reinigung von Kontaktlinsen, wobei die Kontaktlinse in der Zusammensetzung nach einem der Ansprüche 1 bis 12 mindestens 1 Minute lang aufbewahrt wird.

18. Verwendung einer Zusammensetzung nach einem der Ansprüche 1 bis 12 zur täglichen oder periodischen Reinigung, chemischen Desinfektion, Aufbewahrung und Spülung von Kontaktlinsen.

19. Kontaktlinsen-Pflegezusammensetzung, umfassend Miltefosin als antimikrobielles Mittel zur Desinfektion von Kontaktlinsen, vorzugsweise in einer Menge von 5-500 µM, insbesondere 20-200 µM, und eine Protease, wobei die Zusammensetzung vorzugsweise weiters nach einem der Ansprüche 4 bis 9, 11 und 12 definiert ist.

20. Set, enthaltend eine Zusammensetzung nach Anspruch 19, Spüllösungen und/oder Tropfenabgabe-Werkzeuge, vorzugsweise Pipetten.

## Revendications

1. Utilisation de miltéfosine comme agent anti-microbien dans une composition pour l'entretien des lentilles de contact, à des fins de désinfection de lentilles de contact.

2. Utilisation suivant la revendication 1, dans laquelle la miltéfosine est présente en une quantité de 5 à 500 µM dans la composition.

3. Utilisation suivant la revendication 1 ou 2, dans laquelle la miltéfosine est présente en une quantité de 20 à 200 µM dans la composition.

4. Utilisation suivant l'une quelconque des revendications 1 à 3, **caractérisée en ce que** la composition comprend un constituant servant de tampon, de préférence un tampon au phosphate.

5. Utilisation suivant l'une quelconque des revendications 1 à 4, dans laquelle le pH de la solution de la composition est compris dans la plage de 6 à 8.

6. Utilisation suivant l'une quelconque des revendications 1 à 5, **caractérisée en ce que** la composition comprend en outre un agent tensioactif.

7. Utilisation suivant l'une quelconque des revendications 1 à 6, **caractérisée en ce que** la composition comprend en outre un constituant d'augmentation de viscosité, choisi de préférence dans le groupe comprenant des dérivés de cellulose, des polyols, des polymères contenant du poly(oxyde d'éthylène) (PEO), l'alcool polyvinylique et la polyvinylpyrrolidone.

8. Utilisation suivant l'une quelconque des revendications 1 à 7, **caractérisée en ce que** la composition comprend un constituant de tonicité, de préférence des sels tels que le chlorure de sodium, le chlorure de potassium et le chlorure de calcium, ou des agents de tonicité non-ioniques, de préférence le propylèneglycol ou le glycérol.

9. Utilisation suivant l'une quelconque des revendications 1 à 8, **caractérisée en ce que** la composition comprend un constituant chélatant, de préférence l'acide éthylènediamine-tétra-acétique, des sels de métaux alcalins d'acide éthylènediamine-tétra-acétique ou leurs mélanges.

10. Utilisation suivant l'une quelconque des revendications 1 à 9, **caractérisée en ce que** la composition comprend en outre une protéase.

11. Utilisation suivant l'une quelconque des revendications 1 à 10, **caractérisée en ce que** la composition comprend des désinfectants supplémentaires.

12. Utilisation suivant l'une quelconque des revendications 1 à 11, **caractérisée en ce que** la composition est mise par compression sous forme d'un comprimé.

13. Utilisation d'une composition telle que définie dans l'une quelconque des revendications 1 à 12 pour le nettoyage, la désinfection chimique, le stockage et/ou le rinçage de lentilles de contact.

14. Utilisation suivant la revendication 13, pour l'élimination des organismes protozoaires d'une lentille de contact.

15. Utilisation suivant la revendication 14, pour l'élimination des Acanthamoeba d'une lentille de contact.

16. Utilisation suivant l'une quelconque des revendications 1 à 15, dans laquelle la lentille de contact est choisie entre des lentilles dures, des lentilles souples, des lentilles rigides et des lentilles souples perméables aux gaz, des lentilles souples étant préférées.

17. Méthode de purification de lentilles de contact, dans laquelle la lentille de contact est stockée dans la composition telle que définie dans l'une quelconque des revendications 1 à 12 pendant au moins 1 minute.

18. Utilisation d'une composition telle que définie dans l'une quelconque des revendications 1 à 12 pour un nettoyage quotidien ou périodique, une désinfection chimique, un stockage et un rinçage de lentilles de contact.

19. Composition d'entretien de lentilles de contact, comprenant de la miltéfosine comme agent anti-microbien pour la désinfection de lentilles de contact, de préférence en une quantité de 5 à 500 µM, en particulier de 20 à 200 µM, et une protéase, ladite composition étant de préférence en outre définie de la manière indiquée dans l'une quelconque des revendications 4 à 9, 11 et 12.

20. Kit contenant une composition suivant la revendication 19, des solutions de rinçage et/ou des outils d'administration de gouttes, de préférence des pipettes.
